(19) Europäisches Patentamt
European Patent Office
Office européen des brevets

(11) **EP 4 431 119 B1**

(12) **EUROPÄISCHE PATENTSCHRIFT**

(45) Veröffentlichungstag und Bekanntmachung des Hinweises auf die Patenterteilung:
**05.11.2025 Patentblatt 2025/45**

(21) Anmeldenummer: **23161848.9**

(22) Anmeldetag: **14.03.2023**

(51) Internationale Patentklassifikation (IPC):
**A61L 2/07** (2006.01)   **F22B 35/00** (2006.01)

(52) Gemeinsame Patentklassifikation (CPC):
**A61L 2/07; F22B 1/285; F22D 5/24;** A61L 2202/14

(54) **VERFAHREN ZUR NIVEAUREGELUNG VON SPEISEWASSER IN EINEM DAMPFSTERILISATOR SOWIE DAMPFSTERILISATOR**

METHOD FOR CONTROLLING THE LEVEL OF FEEDWATER IN A STEAM STERILIZER AND STEAM STERILIZER

PROCÉDÉ DE RÉGULATION DU NIVEAU D'EAU D'ALIMENTATION DANS UN STÉRILISATEUR À VAPEUR ET STÉRILISATEUR À VAPEUR

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(43) Veröffentlichungstag der Anmeldung:
**18.09.2024 Patentblatt 2024/38**

(73) Patentinhaber: **MELAG Medizintechnik GmbH & Co. KG**
**10829 Berlin (DE)**

(72) Erfinder:
• **LICHT, Tobias**
**13407 Berlin (DE)**

• **VON DER WAYDBRINK, Eberhard**
**14641 Paulinenaue (DE)**
• **SCHMALZ, Elena**
**12526 Berlin (DE)**

(74) Vertreter: **Maikowski & Ninnemann Patentanwälte Partnerschaft mbB**
**Postfach 15 09 20**
**10671 Berlin (DE)**

(56) Entgegenhaltungen:
**EP-A1- 3 276 068       EP-A2- 0 193 863**
**CN-A- 108 613 160       US-A- 3 828 737**
**US-A- 4 526 136          US-A1- 2014 177 772**
**US-A1- 2020 108 163**

**Beschreibung**

Die Erfindung betrifft ein Verfahren zur Niveauregelung von Speisewasser in einer Kammer eines Dampfsterilisators oder in einem Dampferzeuger mit damit verbundener Kammer eines Dampfsterilisators nach dem Oberbegriff des Anspruchs 1 sowie ein Dampfsterilisator nach dem Oberbegriff des Anspruchs 14.

Bei der Dampfsterilisation wird mittels Wasserdampf eine Sterilisation erreicht. Dazu muss der Wasserdampf in der Sterilisationskammer eine bestimmte Temperatur-Druck-Charakteristik über eine definierte Zeitdauer aufweisen, um sicher zu stellen, dass eine Sterilisation von in der Sterilisationskammer befindlichem Ladegut auch tatsächlich erreicht wird. Der Wasserdampf wird durch Verdampfen von, insbesondere vollentsalztem, Speisewasser erzeugt. Hierzu wird Speisewasser direkt in der Sterilisationskammer erhitzt und verdampft oder über einen externen Dampferzeuger der Sterilisationskammer zugeleitet. Um einen solchen Dampfsterilisator energieeffizient zu betreiben, sollte die Speisewassermenge im externen Dampferzeuger bzw. in der Kammer optimal gewählt sein. Sie sollte so gering wie möglich sein, aber gleichzeitig ausreichend, um eine Überhitzung des Heizelements und damit eine vorzeitige Degradation desselben sowie Störungen im Betrieb zu vermeiden.

Hierzu sind aus dem Stand der Technik verschiedene Verfahren zur Regelung des Speisewasserniveaus bekannt. Diese greifen entweder auf den Durchfluss, den Füllstand oder auf die Temperatur zurück, um ein möglichst optimales Speisewasserniveau über den gesamten Sterilisationsprozess zu gewährleisten.

So beschreibt beispielsweise die DE 102 60 895 A1 den Einsatz von Durchflusssensoren zur Speisewasserkennung. Je nach Funktionsprinzip kommen hier unterschiedliche Komponenten zum Einsatz, wie beispielsweise Messturbinen, Vortex-Durchfluss- oder Ultraschalldurchflusssensoren, Strömungswächter oder Differenzdrucksensoren. Diese zusätzlichen Komponenten erhöhen die Kosten und sind eine zusätzliche Fehlerquelle.

Auch die beispielsweise in DE 10 2009 044 053 A1 beschriebene Füllstandsüberwachung erfordert zusätzliche Komponenten mit dem entsprechenden Kostennachteil.

Eine Füllstandsüberwachung mittels Leitfähigkeitsmessung wie in EP 383 327 A1 beschrieben, ist aufgrund der niedrigen Leitfähigkeit des bei der Sterilisation verwendeten vollentsalzten Wassers auf die Dampfsterilisation nicht anwendbar.

Auch mittels Temperaturmessung lässt sich auf den Füllstand rückschließen. So beschreibt EP 193 863 A2 die Bestimmung des Füllstands mittels eines Kapillarrohrreglers. Hierbei wird für jeden Füllstand ein Temperaturfühler in der Nähe des zugehörigen Heizelements angeordnet, der Bestandteil eines mit einer Ausdehnungsflüssigkeit gefüllten Schaltsystems ist. Nachteilig hierbei ist, dass derlei Systeme nur einen festen Temperaturschaltpunkt zulassen sowie eine Hysterese in Bezug auf die Unterschreitung der mit dem Temperaturschaltpunkt assoziierten Temperatur aufweisen.

Auch EP 1 108 384 B1 nutzt die Temperaturmessung, hier mit einem auf bzw. in der Nähe des Heizelements angeordneten PTC-Widerstands, zur Füllstandsbestimmung.

Aus der DE 60 004 509 T2 ist beispielsweise eine Regelung des Speisens in Abhängigkeit von der Temperatur des Heizelements bekannt. Auch mit solchen temperaturgesteuerten Niveauregelungen sind Nachteile verbunden. In der Regel wird bei solchen temperaturgesteuerten Regelungen ein Speisevorgang durch Überschreitung eines festgelegten Temperaturgrenzwerts eingeleitet. Mit einer solchen Regelung sind diverse Nachteile verbunden. Zunächst müssen solche Temperaturgrenzwerte naturgemäß, in der Druckaufbauphase, oberhalb der für die Druckaufbauphase notwendigen Temperatur (z.B. 120°C) und, in der Sterilisationsphase, sogar oberhalb der Sterilisationstemperatur (z.B. 134°C) liegen. Nur so kann ein auf den Bedarfsfall beschränktes Nachspeisen sichergestellt werden. Um einen störungssicheren Betrieb, insbesondere auch unter realen, nicht immer idealen Bedingungen, sicherzustellen, ist es notwendig, den Temperaturgrenzwert höher zu wählen. Beispielsweise verschlechtern Ablagerungen, insbesondere auf einem innerhalb des Dampferzeugers oder in der Sterilisationskammer angeordneten Heizelement, die Wärmeübertragung vom Heizelement auf das Speisewasser. Dies ist insbesondere der Fall, wenn aus Kostengründen oder mangelnder Verfügbarkeit, kein vollentsalztes Wasser als Speisewasser verwendet wird. Erfahrungsgemäß weisen in eine Kammer eingebaute Heizelemente auch im Laufe des Betriebes starke Ablagerungen auf, bestehend aus Inhaltsstoffen aus Pflegeölen für Hand- und Winkelstücke im Dentalbereich, Pflegemitteln für chirurgisches Instrumentarium und Ablagerungen aufgrund nicht fachgerechter Pflege. Temperaturgrenzwerte zwischen 160°C bis 200°C können daher im Betrieb zu fehlerhaft ausgelösten Speisevorgängen führen. In der Praxis haben sich erst deutlich höhere Grenzwerte von bis zu 210 °C bewährt. Selbst mit diesen hohen Grenzwerten kann es zu einem unerwünschten Auslösen eines Überhitzungsschutzschalters kommen. Da der Überhitzungsschutzschalter eine Sicherheitskomponente darstellt und das Heizelement von der Steuerung trennt und erst durch manuelles Betätigen wieder rückstellbar ist, sollte jede unnötige Auslösung des Überhitzungsschutzschalters vermieden werden.

Demgegenüber beschreibt die EP 3 276 068 A1 einen Dampferzeuger für eine Waschmaschine.

Nachteilig ist auch, dass ein hoher Temperaturgrenzwert zu einer längeren und stärkeren Erhitzung des Heizelements führt. Durch Trockenheizen kommt es verstärkt zu Ablagerungen, beispielsweise auf einem innerhalb des Dampferzeugers oder in der Sterilisationskammer angeordneten Heizelements, so dass sich die Störanfälligkeit verschärft. Auch lässt sich mit festen Temperaturgrenzwerten kein wirksamer Überhitzungsschutz schaffen. Denn aufgrund einer durch verlängerte Ansprechzeiten und entsprechend auch verlängerte Abkühlzeiten verursachten Dynamik kann eine Überhitzung des Heizelements trotz Nachspeisens unter Umständen nicht verhindert werden. Häufige und starke Erhitzung sowie Überhitzung des Heizelements, verkürzen dessen Lebensdauer und beeinträchtigen seine Funktionstüchtigkeit jedoch nachteilig.

Vor diesem Hintergrund stellt sich die vorliegende Erfindung die Aufgabe, ein Verfahren zur Niveauregelung von Speisewasser in einer Kammer eines Dampfsterilisators oder in einem Dampferzeuger mit damit verbundener Kammer eines Dampfsterilisators, sowie einen entsprechenden Dampfsterilisator zur Verfügung zu stellen, unter deren Einsatz die aus dem Stand der Technik bekannten Nachteile vermieden werden. Insbesondere ist es Aufgabe der vorliegenden Erfindung, eine kostengünstige Niveauregelung zur Verfügung zu stellen, die auf zusätzliche Komponenten verzichtet, aber trotzdem eine Minimierung der Speisewassermenge und damit des Energiebedarfs und der Programmlaufzeiten erlaubt, sowie eine für die Lebensdauer der Heizelemente negative, regelmäßige Überhitzung wirksam verhindert und die Dampfqualität, insbesondere durch die mit einer Minimierung der Speisewassermenge einhergehende Senkung eines Gehalts an nichtkondensierbaren Gasen in der Kammer bzw. in dem mit einer Kammer verbundenen Dampferzeuger, zu verbessern.

Diese Aufgabe wird durch ein Verfahren zur Niveauregelung von Speisewasser in einer Kammer eines Dampfsterilisators oder in einem Dampferzeuger mit damit verbundener Kammer eines Dampfsterilisators mit den Merkmalen des Anspruch 1 gelöst. Ein solches Verfahren sieht vor, dass das Speisewasser mittels eines Heizelements erhitzt und damit Wasserdampf für die Kammer erzeugt wird. Das Verfahren zeichnet sich erfindungsgemäß dadurch aus, dass eine Temperaturänderungsrate des Heizelements ermittelt wird und ein Nachspeisen, insbesondere der Kammer oder des Dampferzeugers, mit Speisewasser in Abhängigkeit von der ermittelten Temperaturänderungsrate des Heizelements erfolgt.

Hierdurch wird ein echt bedarfsgerechtes Speisen ermöglicht. Aufgrund der Änderungsraten-basierten Steuerung wird ein drohendes Überhitzen deutlich schneller erkannt als bei einer Steuerung auf Basis vorgegebener Temperaturgrenzwerte. Es ist somit möglich, die Speisewassermenge auf das notwendige Minimum zu begrenzen und gleichzeitig ein ständig wiederkehrendes Überhitzen des Heizelements zu vermeiden.

Das erfindungsgemäße Verfahren findet sowohl Anwendung bei der Niveauregelung in Dampfsterilisatoren mit interner Dampferzeugung, bei denen die Dampferzeugung direkt in einer Kammer des Sterilisators selbst erfolgt. Die Kammer ist beispielsweise eine Sterilisationskammer zur Aufnahme von zu sterilisierendem Ladegut mittels Wasserdampf. Bei der internen Dampferzeugung fungiert die Kammer, insbesondere die Sterilisationskammer, gleichzeitig auch als Kammer zur Erzeugung von Wasserdampf aus Speisewasser. Hierzu ist ein Heizelement vorgesehen, das beispielsweise in der Kammer angeordnet ist. Im Bereich des Heizelements kann eine Aufnahme für Speisewasser von der übrigen Kammer abgetrennt sein. Das erfindungsgemäße Verfahren ist aber ebenso auf Dampfsterilisatoren mit externer Dampferzeugung anwendbar. Hier wird der Wasserdampf nicht in einer Kammer, insbesondere Sterilisationskammer, des Sterilisators selbst, sondern in einem externen Dampferzeuger hergestellt und der Kammer, insbesondere der Sterilisationskammer, zugeführt. Sterilisationskammer und Dampferzeuger sind also verschieden, aber miteinander in Fluidverbindung. Der Dampferzeuger kann auch als Dampfkessel, insbesondere im Sinne der EN 14222, bezeichnet werden.

Die erfindungsgemäße Niveauregelung kommt beispielsweise bei einem fraktioniertem Vakuumverfahren zum Einsatz. Das fraktionierte Vakuumverfahren ist ein Entlüftungsverfahren, bei dem über wiederholte Fraktionierungen die Sterilisationsbedingungen hergestellt werden. In der Regel sind zwei bis vier Fraktionierungen vorgesehen. Nach einer initialen Druckaufbau- und Evakuierungsphase wechseln sich Druckaufbauphasen und Evakuierungsphasen ab, um eine immer höhere Luftverdünnung zu erzielen. Die Steuerung der Druckaufbau- und Evakuierungsphasen erfolgt üblicherweise mittels vorgegebener Druckwendepunkte bis zur Erreichung eines Zieldrucks der erwünschten Sterilisationstemperatur. Es folgt die Sterilisationsphase mit geregelter Temperatur, sowie ein Druckablass mit anschließender Trocknungsphase.

Speisewasser umfasst insbesondere demineralisiertes, d.h. vollentsalztes, oder destilliertes Wasser. Im Sinne der vorliegenden Erfindung bezeichnet eine Temperaturänderungsrate eine Änderung der Temperatur pro Zeiteinheit, beispielsweise in K/s. Die Temperaturänderungsrate wird in einer Variante als zeitliche Ableitung der Temperatur ermittelt. Die Ermittlung einer Temperaturänderungsrate umfasst sowohl die unmittelbare Messung einer Temperaturänderungsrate als auch die mittelbare Ermittlung einer Temperaturänderungsrate aus gemessenen Temperaturen und Zeitintervallen. Beispielsweise wird die Änderungsrate als Differenz eines zu einem ersten Zeitpunkt gemessenen Temperaturwerts und eines zu einem zweiten, von dem ersten verschiedenen Zeitpunkt gemessenen Temperaturwerts ermittelt und durch das Zeitintervall zwischen erstem und zweiten Zeitpunkt geteilt und so die Temperaturänderungsrate ermittelt werden. In einer Ausgestaltung beträgt das zur Ermittlung der Temperaturänderungsrate genutzte Zeitintervall zwischen 0,1 s und 5 s, insbesondere 1 s. In einer alternativen Ausgestaltung wird die Temperaturänderungsrate basierend auf mehr als zwei Temperaturwerten, insbesondere basierend auf über einen Zeitraum gemittelte Temperaturwerte, gebildet. In einer Variante wird die Änderungsrate mittels Integralbildung berechnet. Die Integralbildung unterdrückt den Rauschanteil der Temperaturmessung. Zur Ermittlung der Temperaturänderungsrate ist beispielsweise ein Temperatursensor im Dampfsterilisator, insbesondere am Heizelement, vorgesehen.

Beim Nachspeisen wird Speisewasser in das von dem Heizelement beheizte Volumen, d.h. die Kammer oder den externen Dampferzeuger, eingebracht.

In einer Variante wird der Beginn des Nachspeisens in Abhängigkeit von der ermittelten Temperaturänderungsrate des Heizelements ausgelöst. Dies ermöglicht ein schnelles Gegensteuern gegen eine drohende Überhitzung sowie die Vermeidung von unnötiger Erhitzung des Heizelements. Während bei einer temperaturgesteuerten Regelung mit einem Temperaturgrenzwert von 210°C ein erwünschter Nachspeisebedarf bei z.B. 100°C ein unnötiges Aufheizen des Heizelements um 110 K nicht verhindert werden kann, führt eine änderungsratengesteuerte Regelung, die durch eine ermittelten Temperaturänderungsrate ausgelöst wird, typischerweise nur zu einem (an sich) überflüssigen Aufheizen um 10 K bis 20 K und nur in Ausnahmefällen um bis zu 30 K.

In einer Variante wird der Beginn des Nachspeisens ausgelöst, wenn die ermittelte Temperaturänderungsrate eine vorgegebene Einschaltänderungsrate überschreitet. Das bedarfsgerechte Nachspeisen wird somit gestartet, wenn die ermittelte Temperaturänderungsrate eine vorgegebene Einschaltänderungsrate überschreitet. In einer Variante umfasst die vorgegebene Einschaltänderungsrate einen festen Einschaltwert. Beispielsweise beträgt der feste Einschaltwert zwischen 0,5K/s und SKIs, insbesondere 2K/s.

In einer Variante wird das Ende des Nachspeisens in Abhängigkeit von der ermittelten Temperaturänderungsrate des Heizelements ausgelöst. Alternativ oder zusätzlich wird das Ende des Nachspeisens in Abhängigkeit von einer ermittelten Druckänderungsrate ausgelöst wird, wobei die Druckänderungsrate eine in der Kammer ermittelte Druckänderungsrate ist. Das Ende des bedarfsgesteuerten Nachspeisens wird somit über die ermittelte Temperaturänderungsrate oder über die ermittelte Druckänderungsrate gesteuert. Das Nachspeisen kann somit auf eine reduzierte Dauer beschränkt werden. Die Speisewassermenge wird somit möglichst gering gehalten, bei gleichzeitiger Vermeidung einer drohenden Überhitzung des Heizelements.

In einer Variante wird das Ende des Nachspeisens ausgelöst, wenn die ermittelte Temperaturänderungsrate des Heizelements eine vorgegebene erste Ausschaltänderungsrate der Temperatur unterschreitet. In einer Variante umfasst die vorgegebene erste Ausschaltänderungsrate der Temperatur einen festen Ausschaltwert der Temperatur. Beispielsweise beträgt der feste Ausschaltwert der Temperatur zwischen -5K/s und 2K/s, insbesondere 1 K/s. Alternativ oder zusätzlich wird das Ende des Nachspeisens ausgelöst, wenn die ermittelte Druckänderungsrate eine vorgegebene Ausschaltänderungsrate des Drucks unterschreitet oder, in einer weiteren Variante, überschreitet. Zusätzlich lässt sich in einer Ausgestaltung aus der ermittelten Druckänderungsrate bei bekannter Leistungsaufnahme des Heizelements auch auf die gespeiste Speisewassermenge rückschließen. In einer Variante umfasst die vorgegebene Ausschaltänderungsrate des Drucks einen festen Ausschaltwert des Drucks. Beispielsweise beträgt der feste Ausschaltwert des Drucks zwischen -5mbar/s und +5mbar/s, insbesondere 0 mbar/s.

In einer Variante wird das bei Auslösung des Endes des Nachspeisens für eine vorgegebene zusätzliche Nachspeisedauer das Nachspeisen fortgesetzt und erst nach Ablauf der zusätzlichen Nachspeisedauer beendet. Die vorgegebene zusätzliche Nachspeisedauer kann beispielsweise durch ein vorgegebenes Zeitintervall bestimmt sein, insbesondere zeitgesteuert erfolgen, oder in Abhängigkeit von der Zeitdauer zwischen dem Auslösen des Beginns des Nachspeisens und dem Auslösen des Endes des Nachspeisens bestimmt werden. Die Bestimmung der zusätzlichen Nachspeisedauer in Abhängigkeit von dem Auslösen des Beginns und des Endes des Nachspeisens, erhöht die Robustheit des Verfahrens. Insbesondere ist es hierdurch möglich, verringerte Fließraten zu berücksichtigen und auszugleichen und so ein vorgegebenes Nachspeisevolumen zu gewährleisten. Das Ende des Auslösens kann in Abhängigkeit von der ermittelten Temperaturänderungsrate oder in Abhängigkeit von der ermittelten Druckänderungsrate erfolgt sein.

In einer Variante erfolgt das Nachspeisen in einer Druckaufbauphase eines Sterilisationsprozesses, insbesondere während einer Druckaufbauphase eines fraktionierten Vakuumverfahrens. Während der Druckaufbauphase kann einmalig oder mehrmalig nachgespeist werden. Eine Druckaufbauphase endet beispielsweise bei Erreichen eines festgelegten Zieldrucks.

In einer Ausgestaltung wird die Einschaltänderungsrate in Abhängigkeit von anderen Steuerungsgrößen, beispielsweise einer gemessenen Temperatur des Heizelements oder einem gemessenen Druck in der Kammer, vorgegeben. Beispielsweise können für unterschiedliche Temperatur- oder Druckbereiche unterschiedliche Werte für die Einschaltänderungsrate vorgegeben werden. So kann für einen ersten Temperaturbereich eine erste Einschaltänderungsrate vorgegeben sein, für einen zweiten Temperaturbereich eine zweite Einschaltänderungsrate und für einen dritten Temperaturbereich eine dritte Einschaltänderungsrate. Der erste Temperaturbereich umfasst beispielsweise Temperaturen unter 80°C, der zweite Temperaturbereich Temperaturen zwischen 80°C und 120°C und der dritten Temperaturbereich Temperaturen zwischen 121°C und 134°C.

In einer alternativen Variante ist die vorgegebene Einschaltänderungsrate eine Funktion der Temperatur, wobei die Funktion insbesondere so gewählt ist, dass sie einem typischen Verlauf einer Temperaturkurve des Heizelements, entspricht. In einer Ausgestaltung ist die vorgegebene erste Ausschaltänderungsrate der Temperatur von der vorgegebenen Einschaltänderungsrate abhängig. Beispielsweise liegt die erste Ausschaltänderungsrate der Temperatur zwischen 0 K/s und 5 K/s, insbesondere 1 K/s, niedriger als die vorgegebene Einschaltänderungsrate.

In einer Variante wird die Temperaturänderungsrate des Heizelements kontinuierlich ermittelt. Die Temperaturänderungsrate wird beispielsweise im Sekundentakt ermittelt. In einer Variante setzt die kontinuierliche Ermittlung der Temperaturänderungsrate ein, wenn eine vorgegebene Temperatur des Heizelements erreicht ist. Diese vorgegebene Temperatur beträgt zum Beispiels zwischen 40°C und 120°C, insbesondere 80°C. Das heißt, ab einer vorgegebenen Temperatur des Heizelements setzt eine Änderungsratenüberwachung ein. Dies ermöglicht einen robusten Regelungsprozess.

In einer Variante wird das Heizelement abgeschaltet, wenn eine obere Grenztemperatur des Heizelements überschritten wird. Diese obere Grenztemperatur liegt beispielsweise zwischen 120°C und 240°C, insbesondere bei 180°. Alternativ wird das Heizelement abgeschaltet, wenn die ermittelte Temperaturänderungsrate des Heizelements eine zweiten Ausschaltänderungsrate der Temperatur überschreitet. Die zweite Ausschaltänderungsrate der Temperatur liegt beispielsweise zwischen 5 K/s und 12 K/s, insbesondere bei 6 K/s. Das Speisen wird hierbei aber kontinuierlich oder diskontinuierlich fortgesetzt. Hiermit ist die Detektion von nicht ausreichendem oder vollständig fehlendem Speisewasser, beispielsweise aufgrund einer defekten oder verschlissenen Speisepumpe oder eines leeren Speisewassertanks, möglich. So wird eine Überhitzung des Heizelements auch in diesem Störungsfall verhindert und verlängert die Lebensdauer des Heizelements und damit auch des zugehörigen Dampfsterilisators.

In einer Variante wird das Heizelement, insbesondere nach ein Abschaltung bei Überschreitung einer oberen Grenztemperatur oder bei Überschreitung der zweiten Ausschaltänderungsrate der Temperatur, eingeschaltet, wenn eine untere Grenztemperatur unterschritten wird. Diese untere Grenztemperatur liegt beispielsweise zwischen 0 K und 80 K, insbesondere 10 K, unterhalb der oberen Grenztemperatur. In einer Variante ist vorgesehen, dass bei einer erneuten Überschreitung der oberen Grenztemperatur oder bei einem erneuten Überschreiten der zweiten Ausschaltänderungsrate der Temperatur, das Verfahren direkt abgebrochen, insbesondere das Heizen und das Speisen beendet, wird. Insbesondere wird ein Sterilisationsverfahren, in dessen Rahmen die Niveauregelung stattfindet, abgebrochen. Alternativ werden die Schritte Einschalten des Heizelements bei Unterschreiten der unteren Grenztemperatur und Ausschalten des Heizelements bei Überschreitung der oberen Grenztemperatur oder Überschreitung der zweiten Ausschaltänderungsrate der Temperatur ein- oder mehrfach wiederholt und das Verfahren dann abgebrochen, insbesondere das Heizen und das Speisen beendet. Insbesondere wird das Sterilisationsverfahren, in dessen Rahmen die Niveauregelung stattfindet, abgebrochen. Dies stellt sicher, dass bei tatsächlichem und im Rahmen der Regelung nicht behebbaren Speisewassermangel oder einem vollständigen Fehlen von Speisewasser, das Sterilisationsverfahren beendet wird.

In einer Variante wird ein initiales Speisen von Speisewasser in die Kammer oder in den Dampferzeuger gestartet, wenn während einer ersten Evakuierungsphase der Kammer ein vorgegebener Druck in der Kammer unterschritten wird. Dieses initiale Speisen kann beispielsweise zeitgesteuert erfolgen. Durch das zeitgesteuerte initiale Speisen wird sichergestellt, dass eine ausreichende Mindestspeisewassermenge, insbesondere zu Beginn eines Sterilisationsverfahrens und vor einer Druckaufbauphase, zur Verfügung steht. Dies ist wichtig, um eine Überhitzung des Heizelements zu verhindern. Das initiale Speisen, insbesondere während einer ersten Evakuierungsphase der Kammer, kann aber auch über die Temperatur des Heizelements und die ermittelte Temperaturänderungsrate geregelt werden. In einer Variante wird das initiale Speisen von Speisewasser in die Kammer oder in den Dampferzeuger, insbesondere während einer ersten Evakuierungsphase der Kammer, gestartet, wenn das Heizelement eine vorgegebene Regeltemperatur erreicht. Beispielsweise beträgt die vorgegebene Regeltemperatur zwischen 40°C und 100°C, insbesondere 80°C. Das initiale Speisen wird beispielsweise beendet, wenn die ermittelte Temperaturänderungsrate eine vorgegebene dritte Ausschaltänderungsrate der Temperatur erreicht hat. Auch auf diese Weise lässt sich eine Mindestspeisewassermenge sicherstellen und so eine Überhitzung des Heizelements verhindern.

Die der Erfindung zugrunde liegende Aufgabe wird auch durch einen Dampfsterilisator nach Anspruch 14 gelöst. Ein solcher Dampfsterilisator weist ein Heizelement zur Erhitzung von Speisewasser auf. Das zur Erhitzung vorgesehene Speisewasser befindet sich direkt in einer Kammer des Dampfsterilisators oder in einem zur externen Dampferzeugung vorgesehenen Dampferzeuger des Dampfsterilisators. Der Dampferzeuger ist mit einer Kammer des Dampfsterilisators verbunden, so dass im Dampferzeuger generierter Wasserdampf in die Kammer gelangen kann.

Der erfindungsgemäße Dampfsterilisator zeichnet sich dadurch aus, dass er eingerichtet ist, im Betrieb ein Verfahren mit den Merkmalen der Ansprüche 1 bis 13 auszuführen.

Somit wird ein Dampfsterilisator zur Verfügung gestellt, der ein echt bedarfsgerechtes Speisen und damit einen energieeffizienten Betrieb ermöglicht. Der Dampfsterilisator ist weniger störanfällig und weist eine längere Lebensdauer auf, da die Lebensdauer des Heizelements durch die geringere Anzahl von Überhitzungen verlängert wird.

Wie in Bezug auf das erfindungsgemäße Verfahren beschrieben, umfasst der erfindungsgemäße Dampfsterilisator Sterilisatoren mit interner und Sterilisatoren mit externer Dampferzeugung. Das Heizelement kann innerhalb der Kammer oder des Dampferzeugers, insbesondere im Bereich des Bodens, in einer Wand der Kammer oder des Dampferzeugers oder außerhalb der Kammer oder des Dampferzeugers, aber in Kontakt mit der Kammer oder dem Dampferzeuger angeordnet sein.

In einer Variante ist das Heizelement als Rohrheizkörper ausgebildet und am Boden der Kammer des Dampfsterilisators mit interner Dampferzeugung angeordnet.

Der Dampfsterilisator weist in einer Variante einen Temperatursensor zur Ermittlung einer Temperaturänderungsrate auf. Der Temperatursensor ist beispielsweise so angeordnet, dass er den Punkt der stärksten Temperaturänderung erfasst. In einer Variante ist das Heizelement am Boden der Kammer bzw. des Dampferzeugers und der Temperatursensor auf einer Oberseite des Heizelements angeordnet. Dabei bezeichnet Oberseite die dem Boden abgewandte und bei Sinken des Speisewasserniveaus zuerst trocken liegende Seite des Heizelements. Hierdurch wird die Ansprechzeit verkürzt.

In einer Variante umfasst der Dampfsterilisator eine Software, die, wenn sie auf einem Prozessor des Dampfsterilisators ausgeführt wird, den Prozessor dazu veranlasst, ein Verfahren gemäß einem der Ansprüche 1 bis 13 auszuführen. Insbesondere setzt die Software ein computerimplementiertes Verfahren mit den Merkmalen der Ansprüche 1 bis 13 um. Diese Implementierung stellt eine besonders einfach umzusetzende, nachzurüstende und kostengünstige Umsetzung des erfindungsgemäßen Verfahrens dar.

Sämtliche Varianten und Ausgestaltungen des Verfahrens können in beliebiger Weise miteinander kombiniert werden und können entweder einzeln oder in beliebiger Kombination auf den Dampfsterilisator übertragen werden. In gleicher Weise können sämtliche Varianten und Ausgestaltungen des Dampfsterilisators in beliebiger Weise miteinander kombiniert werden und einzeln oder in beliebiger Kombination auf das Verfahren übertragen werden.

Einzelheiten von Aspekten der vorliegend beanspruchten Erfindung werden nachfolgend anhand von Ausführungsbeispielen und Figuren näher erläutert. Es zeigen:

Figur 1   eine Darstellung der Ermittlung einer Temperaturänderungsrate gemäß einer Ausführungsform der vorliegenden Erfindung;

Figur 2   eine Darstellung der Einschalt- und Ausschaltänderungsraten gemäß einer Ausführungsform der vorliegenden Erfindung;

Figur 3   eine Darstellung eines Verfahrens gemäß einer Ausführungsform, insbesondere eines mittels einer Temperaturänderungsrate gestartetes und beendetes Nachspeisen der vorliegenden Erfindung;

Figur 4   eine Darstellung eines Verfahrens gemäß einer Ausführungsform, insbesondere eines änderungsratengesteuerten Speisens in Druckaufbauphasen der vorliegenden Erfindung;

Figur 5   eine Darstellung eines Dampfsterilisators gemäß einer Ausführungsform der vorliegenden Erfindung; und

Figur 6   ein Heizelement mit Temperatursensor gemäß einer Ausführungsform der vorliegenden Erfindung.

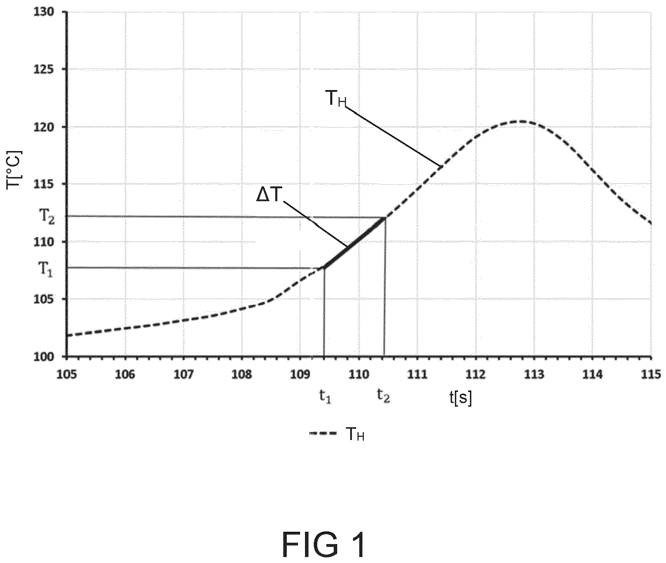

Die Figur 1 illustriert die Ermittlung einer Temperaturänderungsrate ΔT, d.h. einer Änderung der Temperatur $T_H$ des Heizelements 4 pro Zeiteinheit, eines Heizelements 4 eines Dampfsterilisators 1 gemäß einer Ausführungsform der vorliegenden Erfindung. Die Temperaturänderungsrate ΔT wird als gemessene Änderung der Temperatur $T_H$ des Heizelements 4 pro Zeiteinheit ermittelt. Sie entspricht damit der Steigung der Temperaturverlaufskurve $T_H(t)$. Zum Zeitpunkt $t_i$ ist die ermittelte Temperaturänderungsrate ΔT in der vorliegenden Ausführungsform gegeben durch:

$$\Delta T(t_i) = \frac{T(t_i) - T(t_{i-1})}{t_i - t_{i-1}}.$$

Die Änderungsrate ΔT wird fortlaufend in gegebenen Zeitintervallen ermittelt, beispielsweise im Sekundentakt. Um einen kontinuierlichen Verlauf der Temperaturänderungsrate ΔT zu erzielen, kann die Temperaturänderungsrate ΔT auch fortlaufend gemittelt werden. Dabei ist die Mittelung so zu wählen, dass die dem Sterilisationsprozess zugrundeliegende Dynamik sichtbar bleibt und nicht unterdrückt wird.

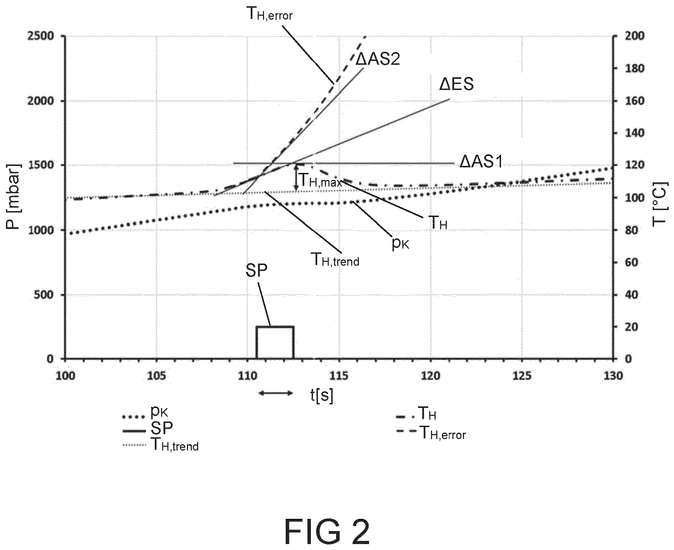

In Figur 2 ist eine Niveauregelung für Speisewasser in einer Kammer 2 bzw. in einem mit einer Kammer 2 verbundenen Dampferzeugers eines Dampfsterilisators gemäß einer Ausgestaltung dargestellt. Figur 2 zeigt den zeitlichen Verlauf der Temperatur $T_H$ des Heizelements 4 sowie den zeitlichen Verlauf des Drucks $p_K$ in der Kammer 2. Die Temperaturänderungsrate ΔT des Heizelements 4 wird, wie beispielsweise in Bezug auf Figur 1 beschrieben, ermittelt. Ein Nachspeisen von Speisewasser erfolgt dann in Abhängigkeit von der ermittelten Temperaturänderungsrate ΔT. Dabei wird das Speisewasser im Falle einer externen Dampferzeugung in den externen Dampferzeuger bzw. im Falle einer internen Dampferzeugung direkt in die Kammer 2 gespeist. Beispielsweise wird der Beginn des Nachspeisens in Abhängigkeit von der Temperaturänderungsrate ∆T ausgelöst. Dies ermöglicht ein echtes bedarfsgerechtes Nachspeisen, insbesondere ein schnelles Reagieren auf Temperaturabweichungen. Beispielsweise ist in der vorliegenden Ausgestaltung eine Einschaltänderungsrate ∆ES vorgegeben. Das Nachspeisen wird gestartet, wenn die ermittelte Temperaturänderungsrate ∆T die vorgegebene Einschaltänderungsrate ∆ES überschreitet. Die vorgegebene Einschaltänderungsrate ∆ES umfasst beispielsweise einen festen Wert. Dieser Wert liegt zum Beispiel zwischen 0,5 K/s und 5 K/s, insbesondere bei 2K/s. Weicht der Temperaturverlauf $T_H$ von der Trendlinie $T_{H,trend}$ des Temperaturverlaufs ab, droht eine Überhitzung des Heizelements 4. Durch das in Abhängigkeit von der ermittelten Temperaturänderungsrate ∆T einsetzende Nachspeisen wird diese Überhitzung abgefangen. Der Temperaturanstieg flacht ab. Das Nachspeisen wird beispielsweise auch in Abhängigkeit von der ermittelten Temperaturänderungsrate ∆T beendet. Hierzu ist zum Beispiel eine erste Ausschaltänderungsrate ∆AS1 der Temperatur vorgegeben, bei dessen Unterschreiten das Nachspeisen beendet wird. Die erste vorgegebene Ausschaltänderungsrate ∆AS1 der Temperatur umfasst vorliegend einen festen Wert, beispielsweise zwischen -5 K/s und 2 K/s, insbesondere 1 K/s. Eine erste vorgegebene Ausschaltänderungsrate ∆AS1 von 0 K/s bewirkt ein Beenden des Nachspeisens bei einem Extremwert, vorliegend einem Maximum $T_{H,max}$, des Temperaturverlaufs $T_H$. Das Nachspeisen wird somit beendet, sobald die Temperatur $T_H$ von einem Temperaturanstieg zu einem Temperaturabfall wechselt. Die beim Nachspeisen zugeführte Speisewassermenge wird so minimiert. Hierdurch wird Energie gespart und Programmlaufzeiten verkürzt. Der Nachspeisevorgang lässt sich am Schaltzustand SP der Speisepumpe 7 im dargestellten Diagramm ablesen.

Um ein Nachspeisen in Abhängigkeit von der ermittelten Temperaturänderungsrate ∆T zu ermöglichen, wird der Temperaturgradient ∆T kontinuierlich ermittelt, d.h. die Temperaturänderungsrate ∆T kontinuierlich überwacht. Die Überwachung der Temperaturänderungsrate ∆T setzt beispielsweise ein, sobald eine vorgegebene Temperatur des Heizelements 4 von beispielsweise zwischen 40°C und 120°C, insbesondere 80°C, erreicht ist. So wird ein robuster Regelungsprozess zur Verfügung gestellt.

In einer Ausgestaltung (nicht dargestellten) wird das Nachspeisen um eine vorgegebene zusätzliche Nachspeisedauer verlängert. Das heißt, dass bei Auslösung des Endes des Nachspeisens, das Nachspeisen nicht sofort beendet wird, sondern darüber hinaus noch für die vorgegebene zusätzliche Nachspeisedauer fortgesetzt wird. Erst nach Ablauf der zusätzlichen Nachspeisedauer wird das Nachspeisen beendet. Hierdurch wird eine ausreichende Speisewassermenge am Ende des Nachspeisevorgangs sichergestellt. Die zusätzliche Nachspeisedauer ist beispielsweise durch ein vorgegebenes Zeitintervall bestimmt (zeitgesteuertes zusätzliches Nachspeisen). Alternativ wird die zusätzliche Nachspeisedauer in Abhängigkeit von der Zeitdauer zwischen dem Auslösen des Beginns des Nachspeisens und dem Auslösen des Endes des Nachspeisens bestimmt.

Ist aufgrund eines Fehlerfalls, beispielsweise einer verschlissenen oder defekten Speisepumpe 7 oder eines leeren Speisewassertanks, ein Nachspeisen gar nicht oder nur unvollständig möglich, kann eine Überhitzung des Heizelements 4 durch Nachspeisen nicht verhindert werden, wie sich an dem Temperaturverlauf $T_{H,error}$ für den Fehlerfall ablesen lässt. Für diesen Fall ist eine zweite Ausschaltänderungsrate ∆AS2 der Temperatur vorgesehen, bei dessen Überschreiten das Heizelement 4 abgeschaltet wird. Die zweite Ausschaltänderungsrate ∆AS2 der Temperatur beträgt beispielsweise zwischen 5 K/s und 12 K/s, insbesondere 6 K/s. Das Speisen wird hierbei jedoch, kontinuierlich oder diskontinuierlich, fortgesetzt. Auf diese Weise kann eine unzureichende Speisewassermenge erkannt und einer Überhitzung des Heizelements 4 im Fehlerfall vorgebeugt werden. Alternativ kann anstelle einer zweiten Ausschaltänderungsrate ∆AS2 auch eine obere Grenztemperatur (nicht dargestellt) vorgesehen sein, so dass das Heizelement 4 abgeschaltet wird, sobald die obere Grenztemperatur überschritten wird. Beispielsweise liegt die obere Grenztemperatur zwischen 120°C und 240°C, insbesondere bei 180°C. Nach einer Abschaltung des Heizelements, aufgrund eines Überschreitens der zweiten Ausschaltänderungsrate ∆AS2 der Temperatur oder aufgrund eines Überschreitens der oberen Grenztemperatur, wird das Heizelement 4 wieder eingeschaltet, sobald eine untere Grenztemperatur (nicht dargestellt) unterschritten wird. Diese untere Grenztemperatur liegt beispielsweise zwischen 0 K und 80 K, insbesondere bei 10 K, niedriger als die obere Grenztemperatur. Wird dann die obere Grenztemperatur oder die zweite Ausschaltänderungsrate ∆AS2 der Temperatur erneut überschritten, wird das Sterilisationsverfahren, insbesondere das Heizen und Speisen, abgebrochen. Alternativ werden die Schritte des Einschaltens bei Unterschreitung der unteren Grenztemperatur und des Ausschaltens bei Überschreitung der oberen Grenztemperatur ein- oder mehrfach wiederholt und dann das Sterilisationsverfahren abgebrochen, insbesondere das Heizen und das Speisen beendet. Dies stellt einen Programmabbruch bei nicht-behebbarem Fehlerfall sicher.

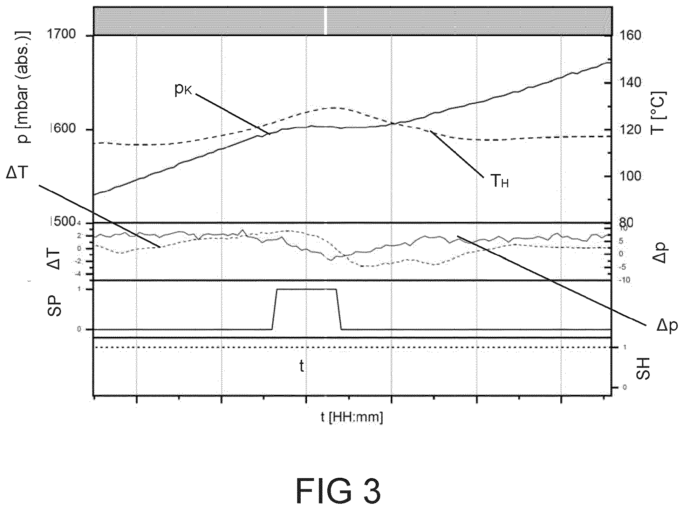

Figur 3 illustriert ein Verfahren zur Niveauregelung von Speisewasser gemäß einer weiteren Ausgestaltung. Es finden die in Bezug auf Figur 1 und 2 erläuterten Verfahrensschritte Anwendung. Dargestellt sind hier neben dem Temperaturverlauf $T_H$ und dem Druckverlauf $p_K$ auch der zeitliche Verlauf der ermittelten Temperaturänderungsrate ∆T, sowie der zeitliche Verlauf der Druckänderungsrate ∆p in der Kammer 2 während einer Druckaufbauphase eines Sterilisationsprozesses, insbesondere eines fraktionierten Vakuumverfahrens. Entsprechend steigt der Druck $p_K$ an; der ermittelte Druckgradient ∆p ist positiv. Die Temperatur $T_H$ des Heizelements 4 ist so gewählt, das in einer Kammer 2 bzw. in einem Dampferzeuger eines Dampfsterilisators 1 befindliches Speisewasser verdampft wird. Entsprechend ist die ermittelte Temperaturänderungsrate ∆T nahezu konstant. Kommt es aufgrund einer zu geringen Speisewassermenge zu einer Temperaturänderung am Heizelement 4 weicht die ermittelte Temperaturänderungsrate ∆T von Null ab. Überschreitet die ermittelte Temperaturänderungsrate ∆T eine vorgegebenen Einschaltänderungsrate ∆ES, wird ein Nachspeisen gestartet. Die Temperaturerhöhung wird abgefangen. Die Temperaturkurve $T_H$ flacht ab. Entsprechend nimmt die ermittelte Temperaturänderungsrate ∆T ab. Unterschreitet er eine vorgegebene erste Ausschaltänderungsrate ∆AS1 der Temperatur, wird das Nachspeisen beendet. Die Temperatur $T_H$ findet zu ihrem Sollwert (hier 100°C) zurück. Der Schaltzustand SP der Speisepumpe 7 zeigt den Beginn und Ende des Nachspeisens, ausgelöst durch die ermittelte Temperaturänderungsrate ∆T.

Wie aus der dargestellten Druckkurve $p_K$ ersichtlich ist, weist der zeitliche Druckverlauf ein Maximum auf. Ist nicht genügend Speisewasser vorhanden, kann der Druck $p_K$ in der Kammer 2 nicht weiter ansteigen. Wird dann, in Abhängigkeit von der ermittelten Temperaturänderungsrate ∆T, ein Nachspeisen eingeleitet, muss das neu eingespeiste Wasser zunächst erhitzt werden, bevor dessen Verdampfung einsetzt. Erst dann beginnt der Druck $p_K$ wieder zu steigen. Somit kann in einer Ausgestaltung das Nachspeisen auch in Abhängigkeit von einer ermittelten Druckänderungsrate ∆p erfolgen. Die ermittelte Druckänderungsrate ∆p entspricht einer gemessenen Druckänderung in der Kammer 2 pro Zeitintervall. Auch die ermittelte Druckänderungsrate ∆p kann gemittelt werden, um einen kontinuierlichen Verlauf zu erhalten. Unterschreitet die ermittelte Druckänderungsrate ∆p beispielsweise eine vorgegebene Ausschaltänderungsrate ∆ASp des Drucks, so wird das Nachspeisen beendet. Die vorgegebene Ausschaltänderungsrate ∆ASp des Drucks beträgt beispielsweise zwischen -5mbar/s und +5mbar/s, insbesondere 0 mbar/s. Hierdurch wird ein alternatives Kriterium zum Beenden des Nachspeisevorgangs geschaffen.

Das Heizelement 4 bleibt während des gesamten Vorgangs eingeschaltet, wie sich am Schaltzustand SH des Heizelements 4 ablesen lässt.

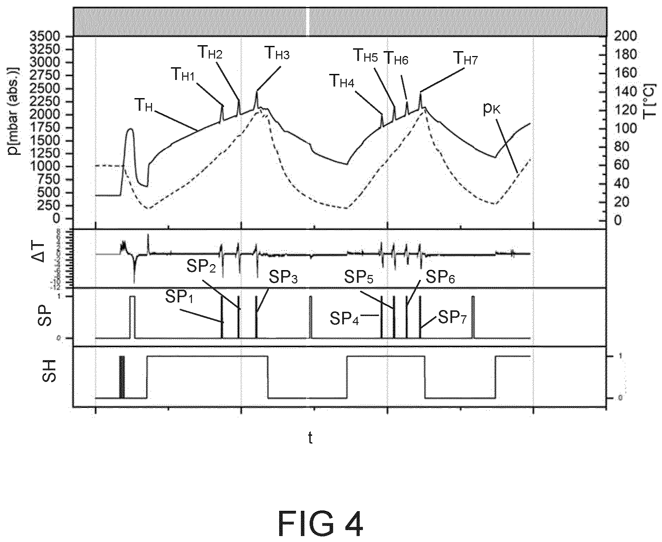

In Figur 4 ist die Niveauregelung gemäß einer Ausgestaltung für mehrere Druckaufbau- und Evakuierungsphasen eines fraktionierten Vakuumverfahrens dargestellt. Es finden die in Bezug auf die Figuren 1 bis 3 erläuterten Verfahrensschritte Anwendung. Wie dargestellt, werden über mehrere sich abwechselnde Druckaufbau- und Evakuierungsphasen, d.h. Fraktionierungen, die Sterilisationsbedingungen (Druck und Temperatur in der Kammer 2) erreicht. Vorliegend dargestellt sind zwei Fraktionierungen, d.h. zwei jeweils von einer Evakuierungsphase gefolgten Druckaufbauphasen. Während der Druckaufbauphase wird der Druck $p_K$ in der Kammer 2, insbesondere durch Verdampfen (und bei einem externen Dampferzeuger zuführen) von Speisewasser, aufgebaut. In der darauffolgenden Evakuierungsphase wird der Wasserdampf durch einen Druckablass abgelassen. Das änderungsrate-basierte Nachspeisen erfolgt in einer Ausgestaltung nur während der Druckaufbauphasen. Deutlich erkennbar sind die drei Temperaturspitzen $T_{H1}$, $T_{H2}$, $T_{H3}$ am Ende der ersten Druckaufbauphase sowie die vier Temperaturspitzen $T_{H4}$, $T_{H5}$, $T_{H6}$, $T_{H7}$ am Ende der zweiten Druckaufbauphase. Die entsprechende Erhöhung der Temperatur $T_H$ des Heizelements 4 wird über die ermittelte Temperaturänderungsrate ∆T detektiert: überschreitet dieser, wie in Bezug auf Figuren 2 und 3 beschrieben, eine vorgegebene Einschaltänderungsrate ∆ES, wird ein Nachspeisevorgang ausgelöst. Dieser wird beendet, wenn die ermittelte Temperaturänderungsrate ∆T eine vorgegebene erste Ausschaltänderungsrate ∆AS1 der Temperatur wieder unterschreitet. Der Nachspeisevorgang kann darüber hinaus um eine zusätzliche Nachspeisedauer fortgesetzt werden. Die entsprechenden Nachspeisevorgänge $SP_1$, $SP_2$, $SP_3$ der ersten Druckaufbauphase sowie die entsprechenden Nachspeisevorgänge $SP_4$, $SP_5$, $SP_6$, $SP_7$ der zweiten Druckaufbauphase sind am Schaltzustand der Speisepumpe ablesbar. Zusätzlich können, wie in Figur 4 erkennbar, prozessbedingte zeitgesteuerte Nachspeisevorgänge erfolgen, auf die vorliegend nicht weiter eingegangen wird. Das Heizelement 4 bleibt während der Druckaufbauphase eingeschaltet, sofern kein Fehlerfall auftritt. Während der Evakuierungsphasen wird das Heizelement 4 abgeschaltet. Das Ab- und Einschalten des Heizelements 4 zwischen den einzelnen Druckaufbau- und Evakuierungsphasen erfolgt in der Regel über Druckschaltpunkte. Die Heizphasen sind am Schaltzustand des Heizelements 4 ablesbar.

Wichtig ist, zu Beginn eines Sterilisationsvorgangs und vor einer Druckaufbauphase eine Mindestspeisewassermenge zur Verfügung zu stellen, um eine Überhitzung des Heizelements 4 zu verhindern. Hierzu sind verschiedene Regelungen eines initialen Speisens möglich. Beginnt das Sterilisationsverfahren mit einer Evakuierungsphase, d.h. ist die erste Phase eine Evakuierungsphase, erfolgt ein initiales Speisen zeitgesteuert, wenn ein vorgegebener Druck in der Kammer 2 unterschritten wird. Ein erfolgreiches initiales Speisen lässt erst sich in der darauffolgenden Druckaufbauphase daran erkennen, dass nicht gleich zu Beginn dieser Phase eine Überhitzung des Heizelements 4 stattfindet. Alternativ erfolgt das initiale Speisen, wenn das Heizelement 4 eine vorgegebene Regeltemperatur erreicht, wobei die vorgegebene Regeltemperatur zwischen 40°C und 100°C, insbesondere 80°C, beträgt. Das initiale Speisen wird beendet, wenn die ermittelte Temperaturänderungsrate ∆T eine vorgegebene dritte Ausschaltänderungsrate ∆AS3 der Temperatur erreicht. Die dritte Ausschaltänderungsrate ∆AS3 der Temperatur ist in einer Ausgestaltung identisch zu der ersten Ausschaltänderungsrate ∆AS1 der Temperatur. Das Verfahren zum initialen Speisen stellt eine Mindestspeisewassermenge zu Beginn eines Sterilisationsverfahrens, insbesondere eines fraktionierten Vakuumverfahrens, sicher.

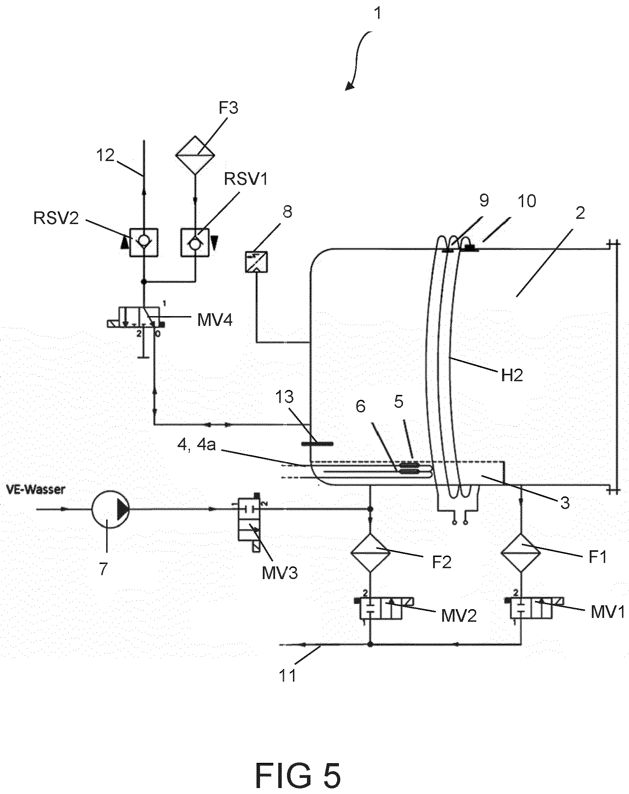

In Figur 5 ist ein Dampfsterilisator 1 gemäß einer Ausführungsform der vorliegenden Erfindung beispielhaft dargestellt. Der Dampfsterilisator 1 zeichnet sich dadurch aus, dass er geeignet und eingerichtet ist, im Betrieb ein Verfahren zur Niveauregelung wie in Bezug auf die vorangehenden Figuren 1 bis 4 erläutert, auszuführen. Hierzu weist der Dampfsterilisator 1 beispielsweise eine Software auf, die, wenn sie auf einem Prozessor ausgeführt wird, selbigen dazu veranlasst ein Verfahren zur Niveauregelung wie in Bezug auf die vorangehenden Figuren 1 bis 4 erläutert, auszuführen.

Der Dampfsterilisator 1 weist ein Heizelement 4 zur Erhitzung von in einer Kammer 2 des Dampfsterilisators 1 oder von einem mit einer Kammer 2 des Dampfsterilisators 1 verbundenen Dampferzeuger des Dampfsterilisators 1 befindlichen Speisewasser auf.

Konstruktiv kann der Dampfsterilisator 1 sowohl eine externe als auch eine interne Dampferzeugung vorsehen. Vorliegend ist ein Dampfsterilisator 1 mit interner Dampferzeugung dargestellt. Das heißt, Speisewasser wird in einer Kammer 2 des Sterilisators 1 direkt verdampft und dort zur Sterilisation von in der Kammer 2 befindlichem Ladegut verwandt. Hierzu ist das Heizelement 4 beispielsweise in der Kammer 2, hier im Bereich 3 eines Bodens der Kammer 2 angeordnet. Das Heizelement 4 ist beispielsweise als Rohrheizkörper 4a ausgeführt. Mittels eines direkt am Rohrheizkörper 4a angebrachten Temperatursensors 5 wird kontinuierlich die Temperaturänderungsrate $\Delta T$ ermittelt. Am Rohrheizkörper 4a ist in einer Variante zusätzlich ein Sicherheitstemperaturbegrenzer 6 angeordnet, der den Rohrheizkörper 4a im Fall einer defekten Steuerung von einer Spannungsversorgung trennt. Die Kammer 2, insbesondere deren Boden, ist in einer Ausgestaltung gegenüber einer Horizontalen geneigt, so dass nur der vordere Teil des Rohrheizkörpers 4a bei Speisewassermangel freigelegt wird.

Die Kammer 2 wird während einer Evakuierungsphase mittels einer Vakuumpumpe, gegebenenfalls mit vorgeschaltetem Dampfkondensator, (beide nicht dargestellt) und gleichzeitigem Öffnen von Magnetventil MV1 evakuiert. Ferner ist in einer Ausgestaltung ein Filter F1 vorgesehen, um das Magnetventil MV1 vor Verunreinigungen, beispielsweise durch gelöste Ablagerungen aus der Kammer, zu schützen. Das Speisen erfolgt mit einer Speisepumpe 7 und gleichzeitigem Öffnen von Magnetventil MV3 von unten in den Bereich 3 der Kammer 2, in dem auch der Rohrheizkörper 4a angeordnet ist.

Der Bereich 3 des Rohrheizkörpers 4a ist vom restlichen Boden der Kammer 2 abgeschottet. Hierdurch wird das benötigte Speisewasser bereits minimiert. Der Dampfaustritt nach oben wird durch Öffnungen, beispielsweise im den Bereich 3 abschottenden Blech, gewährleistet. Gelangt zu viel Speisewasser in den abgeschotteten Bereich 3, beispielsweise nach einem Stromausfall, läuft während des Speisens im nach dem Stromausfall fortgesetzten oder darauf folgenden Sterilisationsverfahren überflüssiges Wasser über die Schottwand in einen vorderen Bereich der Kammer 2, in dem sich auch Kondensat sammeln kann. Dies kann bei einer darauffolgenden Evakuierung abgepumpt werden.

In einer Druckaufbauphase wird Druck durch Einschalten des Rohrheizkörpers 4a aufgebaut. Eine am Umfang der Kammer 2 angeordnete Mantelheizung H2 heizt die Kammer 2 darüber hinaus vor und sorgt für eine gleichmäßige Temperaturverteilung in der Kammer 2 sowie einer Reduktion von in der Kammer 2 anfallendem Kondensat. Die Regelung der Mantelheizung H2 erfolgt vorliegend über einen Temperatursensor 9 der Mantelheizung H2. Auch kann ein Sicherheitstemperaturbegrenzer 10 für die Mantelheizung H2 vorgesehen sein.

Ein im Dampfsterilisator 1 ausgeführtes fraktioniertes Vakuumverfahren kann über einen Drucksensor 8 gesteuert werden, indem ein oberer und ein unterer Druckwendepunkt der Fraktionierungen als Auslöser für den Beginn der Evakuierungs- bzw. der Druckaufbauphase genutzt werden. In der Kammer 2 ist mindestens ein Temperatursensor 13 der Kammer 2 vorgesehen, der die Sterilisationstemperatur verifiziert und/oder zur Steuerung des Verfahrens eingesetzt werden kann.

Der Druckablass und damit auch der Ablass von restlichem Speisewasser und Kondensat am Ende der Sterilisation erfolgt mittels eines Magnetventils MV2, optional über ein weiteres Magnetventil MV1, vorliegend mit davorgeschaltetem Filter F2 bzw. F1.

In der sich anschließende Trocknungsphase erfolgt eine Trocknung mittels Vakuum. Anfallendes Kondensat wird über die Vakuumpumpe abgepumpt. Nach Abschluss der Trocknungsphase wird die Kammer 2 belüftet, indem ein Magnetventil MV4 geöffnet und über einen Sterilfilter sterile Luft durch den in der Kammer 2 befindlichen Unterdruck eingesaugt wird. Ein Rückschlagventil RSV1 verhindert ein Eindringen von Feuchtigkeit aus der Kammer 2 in den Sterilfilter F3.

Im Falle eines Stromausfalls erfolgt ein selbsttätiger Druckablass (Notablass) über das Magnetventil MV4 in einen Abwassertank (nicht dargestellt). Das Rückschlagventil RSV2 verhindert dabei, dass nicht-sterile Luft in die Kammer 2 gelangt.

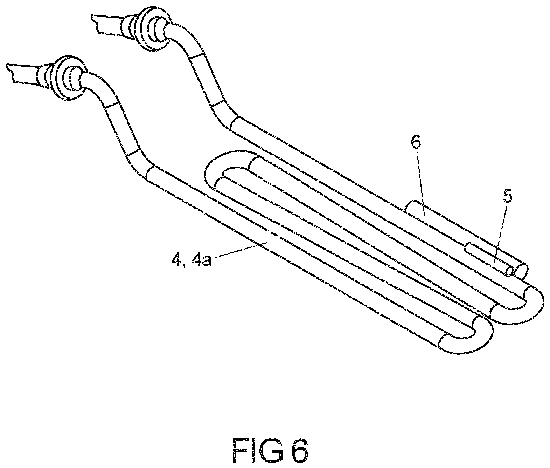

Figur 6 zeigt ein Heizelement 4 zur Erhitzung von in einer Kammer 2 eines Dampfsterilisators 1, beispielsweise der Figur 5, oder in einem mit einer Kammer 2 verbundenen Dampferzeuger eines Dampfsterilisators 1 befindlichen Speisewasser. Das Heizelement 4 ist vorliegend als Rohrheizkörper 4a ausgeführt. Am Rohrheizkörper 4a ist ein Temperatursensor 5 angeordnet. Mittels des Temperatursensors 5 wird eine Temperaturänderungsrate $\Delta T$ ermittelt und in Abhängigkeit von dieser Speisewasser nachgespeist wie in Bezug auf die Figuren 1 bis 4 beschrieben. Beispielsweise ist der Temperatursensor 5 an einer Oberseite des Rohrheizkörpers 4a angeordnet, so dass der Temperatursensor 5 sich in einem sich bei zu niedrigem Speisewasserniveau zuerst erhitzenden Bereich des Rohrheizkörpers 4a befindet. Zusätzlich ist in einer Variante die Kammer 2 gegenüber eine Horizontalen geneigt, so dass nur der vordere Teil des Rohrheizkörpers 4a im Falle von Speisewassermangel freigelegt wird. Ein Sicherheitstemperaturbegrenzer 6 ist ebenfalls am Rohrheizkörper 4a angeordnet. Er dient als zusätzliche Schutzeinrichtung und trennt den Rohrheizkörper 4a von einer Spannungsversorgung im Fall einer defekten Steuerung.

Bezugszeichenliste

| 1 | Dampfsterilisator |
|---|---|
| 2 | Kammer |
| 3 | Bereich |
| 4 | Heizelement |
| 4a | Rohrheizkörper |
| 5 | Temperatursensor |
| 6 | Sicherheitstemperaturbegrenzer |
| 7 | Speisepumpe |
| 8 | Drucksensor |
| 9 | Temperatursensor Mantelheizung |
| 10 | Sicherheitstemperaturbegrenzer Mantelheizung |
| 11 | Zuleitung Vakuumpumpe |
| 12 | Zuleitung Abwassertank |
| 13 | Temperatursensor Kammer |
| F1 | Filter |
| F2 | Filter |
| F3 | Sterilfilter |
| MV1 | Magnetventil |
| MV2 | Magnetventil |
| MV3 | Magnetventil |
| MV4 | Magnetventil |
| RSV1 | Rückschlagventil |
| RSV2 | Rückschlagventil |
| H2 | Mantelheizung |
| $\Delta ES$ | Einschaltänderungsrate |
| $\Delta AS1$ | erste Ausschaltänderungsrate der Temperatur |
| $\Delta AS2$ | zweite Ausschaltänderungsrate der Temperatur |
| $\Delta ASp$ | Ausschaltänderungsrate des Drucks |
| p | Druck (absolut) |
| T | Temperatur |
| t | Zeit |
| $t_i$ | Zeitpunkt i |
| $T_H(t_i)$ | Temperatur Heizelement zum Zeitpunkt $t_i$ |
| $T_{H,trend}$ | Temperatur Heizelement Trendlinie |
| $T_{H,error}$ | Temperatur Heizelement Fehlerfall |
| $T_H$ | Temperatur Heizelement |
| $p_K$ | Druck in Kammer (interne oder externe Dampferzeugung) |
| p(t) | zeitlicher Druckverlauf |
| $\Delta p$ | ermittelte Druckänderungsrate |
| $\Delta T$ | ermittelte Temperaturänderungsrate |
| $T_{H,max}$ | Temperaturmaximum |

| SP | Schaltzustand Speisepumpe |
|---|---|
| SH | Schaltzustand Heizelement |

**Patentansprüche**

1. Verfahren zur Niveauregelung von Speisewasser in einer Kammer (2) eines Dampfersterilisators (1) oder in einem Dampferzeuger mit damit verbundener Kammer (2) eines Dampfsterilisators (1), wobei das Speisewasser mittels eines Heizelements (4, 4a) erhitzt und damit Wasserdampf für die Kammer (2) erzeugt wird,
**dadurch gekennzeichnet,**
**dass** eine Temperaturänderungsrate ($\Delta T$) des Heizelements (4, 4a) ermittelt wird und ein Nachspeisen mit Speisewasser in Abhängigkeit von der ermittelten Temperaturänderungsrate ($\Delta T$) des Heizelements (4, 4a) erfolgt.

2. Verfahren nach Anspruch 1, **dadurch gekennzeichnet, dass** der Beginn des Nachspeisens in Abhängigkeit von der ermittelten Temperaturänderungsrate ($\Delta T$) des Heizelements (4, 4a) ausgelöst wird.

3. Verfahren nach einem der vorhergehenden Ansprüche, **dadurch gekennzeichnet, dass** der Beginn des Nachspeisens ausgelöst wird, wenn die ermittelte Temperaturänderungsrate ($\Delta T$) eine vorgegebene Einschaltänderungsrate ($\Delta ES$) überschreitet.

4. Verfahren nach Anspruch 3, **dadurch gekennzeichnet, dass** die vorgegebene Einschaltänderungsrate ($\Delta ES$) einen festen Einschaltwert umfasst, wobei der feste Einschaltwert zwischen 0,5K/s und 5K/s beträgt.

5. Verfahren nach einem der vorhergehenden Ansprüche, **dadurch gekennzeichnet, dass** das Ende des Nachspeisens in Abhängigkeit von der ermittelten Temperaturänderungsrate ($\Delta T$) des Heizelements (4, 4a) und/oder in Abhängigkeit von einer ermittelten Druckänderungsrate ($\Delta p$) ausgelöst wird, wobei die Druckänderungsrate ($\Delta p$) eine in der Kammer (2) ermittelte Druckänderungsrate ($\Delta p$) ist.

6. Verfahren nach Anspruch 5, **dadurch gekennzeichnet, dass** das Ende des Nachspeisens ausgelöst wird, wenn die ermittelte Temperaturänderungsrate ($\Delta T$) des Heizelements (4, 4a) eine vorgegebene erste Ausschaltänderungsrate ($\Delta AS1$) der Temperatur unterschreitet, wobei die vorgegebene erste Ausschaltänderungsrate ($\Delta AS1$) der Temperatur einen festen Ausschaltwert der Temperatur umfasst, wobei der feste Ausschaltwert der Temperatur zwischen -5K/s und 2K/s beträgt.

7. Verfahren nach Anspruch 5, **dadurch gekenn-**

zeichnet, dass das Ende des Nachspeisens ausgelöst wird, wenn die ermittelte Druckänderungsrate ($\Delta$p) eine vorgegebene Ausschaltänderungsrate ($\Delta$ASp) des Drucks unterschreitet oder das Ende des Nachspeisens ausgelöst wird, wenn die ermittelte Druckänderungsrate ($\Delta$p) eine vorgegebene Ausschaltänderungsrate ($\Delta$ASp) des Drucks überschreitet, wobei die vorgegebene Ausschaltänderungsrate ($\Delta$ASp) des Drucks einen festen Ausschaltwert des Drucks umfasst, wobei der feste Ausschaltwert des Drucks zwischen -5mbar/s und +5mbar/s beträgt.

8. Verfahren nach einem vorhergehenden Ansprüche, **dadurch gekennzeichnet, dass** das bei Auslösung des Endes des Nachspeisens für eine vorgegebene zusätzliche Nachspeisedauer das Nachspeisen fortgesetzt wird und erst nach Ablauf der zusätzlichen Nachspeisedauer beendet wird, wobei die vorgegebene zusätzliche Nachspeisedauer durch ein vorgegebenes Zeitintervall bestimmt ist oder in Abhängigkeit von der Zeitdauer zwischen dem Auslösen des Beginns des Nachspeisens und dem Auslösen des Ende des Nachspeisens bestimmt wird.

9. Verfahren nach einem der vorhergehenden Ansprüche, **dadurch gekennzeichnet, dass** die Temperaturänderungsrate ($\Delta$T) des Heizelements (4, 4a) kontinuierlich ermittelt wird, wobei die kontinuierliche Ermittlung der Temperaturänderungsrate ($\Delta$T) einsetzt, wenn eine vorgegebene Temperatur des Heizelements erreicht ist, wobei die vorgegebene Temperatur zwischen 40°C und 120°C beträgt.

10. Verfahren nach einem der vorhergehenden Ansprüche, **dadurch gekennzeichnet, dass** das Heizelement (4, 4a) abgeschaltet wird, wenn eine obere Grenztemperatur des Heizelements (4, 4a) überschritten wird, wobei die obere Grenztemperatur zwischen 120°C und 240°C beträgt oder wenn die ermittelte Temperaturänderungsrate ($\Delta$T) eine zweite Ausschaltänderungsrate ($\Delta$AS2) der Temperatur überschreitet, wobei die zweite Ausschaltänderungsrate ($\Delta$AS2) der Temperatur zwischen 5 K/s und 12 K/s beträgt, wobei das Speisen kontinuierlich oder diskontinuierlich fortgesetzt wird.

11. Verfahren nach Anspruch 10, **dadurch gekennzeichnet, dass** das Heizelement (4, 4a) eingeschaltet wird, wenn eine untere Grenztemperatur unterschritten wird, wobei die untere Grenztemperatur zwischen 0 K und 80 K unterhalb der oberen Grenztemperatur liegt.

12. Verfahren nach Anspruch 11, **dadurch gekennzeichnet, dass** bei erneutem Überschreiten der oberen Grenztemperatur oder bei erneutem Überschreiten der zweiten Ausschaltänderungsrate ($\Delta$AS2) der Temperatur das Verfahren direkt abgebrochen wird, oder die Schritte des Einschaltens des Heizelements bei Unterschreiten der unteren Grenztemperatur und des Ausschaltens des Heizelements bei Überschreitung der oberen Grenztemperatur oder Überschreitung der zweiten Ausschaltänderungsrate ($\Delta$AS2) der Temperatur ein- oder mehrfach wiederholt werden und das Verfahren dann abgebrochen wird.

13. Verfahren nach einem der vorhergehenden Ansprüche, **dadurch gekennzeichnet, dass** ein initiales Speisen von Speisewasser in die Kammer (2) oder in den Dampferzeuger gestartet wird, wenn während einer ersten Evakuierungsphase der Kammer (2) ein vorgegebener Druck in der Kammer (2) unterschritten wird und/oder wenn das Heizelement (4, 4a) eine vorgegebene Regeltemperatur erreicht, wobei die vorgegebene Regeltemperatur zwischen 40°C und 100°C beträgt, und wobei das initiale Speisen beendet wird, wenn die ermittelte Temperaturänderungsrate ($\Delta$T) eine vorgegebene dritte Ausschaltänderungsrate ($\Delta$AS3) der Temperatur erreicht.

14. Dampfsterilisator (1) mit einer Kammer (2) zur Aufnahme von Speisewasser oder mit einem mit einer Kammer (2) verbundenen Dampferzeuger zur Aufnahme von Speisewasser, wobei der Dampfsterilisator (1) ein Heizelement (4, 4a) zum Erhitzen des Speisewassers aufweist, **dadurch gekennzeichnet, dass** der Dampfsterilisator (1) dafür vorgesehen und eingerichtet ist, im Betrieb ein Verfahren gemäß einem der Ansprüche 1 bis 13 auszuführen.

15. Dampfsterilisator (1) nach Anspruch 14, **dadurch gekennzeichnet, dass** der Dampfsterilisator (1) eine Software umfasst, die, wenn sie auf einem Prozessor des Dampfsterilisators (1) ausgeführt wird, den Prozessor dazu veranlasst, ein Verfahren gemäß einem der Ansprüche 1 bis 13 auszuführen.

## Claims

1. A method for controlling the level of feed water in a chamber (2) of a steam sterilizer (1) or in a steam generator with a chamber (2) of a steam sterilizer (1) connected therewith, wherein the feed water is heated by means of a heating element (4, 4a) and steam thereby is generated for the chamber (2), **characterized in that** a temperature change rate ($\Delta$T) of the heating element (4, 4a) is determined and refeeding with feed water is effected in dependence on the determined temperature change rate ($\Delta$T) of the heating element (4, 4a).

2. The method according to claim 1, **characterized in**

*that* the start of refeeding is triggered in dependence on the determined temperature change rate ($\Delta T$) of the heating element (4, 4a).

3. The method according to any of the preceding claims, **characterized in that** the start of refeeding is triggered when the determined temperature change rate ($\Delta T$) exceeds a specified switch-on change rate ($\Delta ES$).

4. The method according to claim 3, **characterized in that** the specified switch-on change rate ($\Delta ES$) comprises a fixed switch-on value, wherein the fixed switch-on value is between 0.5 K/s and 5 K/s.

5. The method according to any of the preceding claims, **characterized in that** the end of refeeding is triggered in dependence on the determined temperature change rate ($\Delta T$) of the heating element (4, 4a) and/or in dependence on a determined pressure change rate ($\Delta p$), wherein the pressure change rate ($\Delta p$) is a pressure change rate ($\Delta p$) determined in the chamber (2).

6. The method according to claim 5, **characterized in that** the end of refeeding is triggered when the determined temperature change rate ($\Delta T$) of the heating element (4, 4a) falls below a specified first switch-off change rate ($\Delta AS1$) of the temperature, wherein the specified first switch-off change rate ($\Delta AS1$) of the temperature comprises a fixed switch-off value of the temperature, wherein the fixed switch-off value of the temperature is between -5 K/s and 2 K/s.

7. The method according to claim 5, **characterized in that** the end of refeeding is triggered when the determined pressure change rate ($\Delta p$) falls below a specified switch-off change rate ($\Delta ASp$) of the pressure or the end of refeeding is triggered when the determined pressure change rate ($\Delta p$) exceeds a specified switch-off change rate ($\Delta ASp$) of the pressure, wherein the specified switch-off change rate ($\Delta ASp$) of the pressure comprises a fixed switch-off value of the pressure, wherein the fixed switch-off value of the pressure is between -5 mbar/s and +5 mbar/s.

8. The method according to any of the preceding claims, **characterized in that** when the end of refeeding is triggered for a specified additional refeeding period, refeeding is continued and terminated only at the end of the additional refeeding period, wherein the specified additional refeeding period is determined by a specified time interval or is determined in dependence on the time period between the triggering of the start of refeeding and the triggering of the end of refeeding.

9. The method according to any of the preceding claims, **characterized in that** the temperature change rate ($\Delta T$) of the heating element (4, 4a) is determined continuously, wherein the continuous determination of the temperature change rate ($\Delta T$) starts when a specified temperature of the heating element is reached, wherein the specified temperature is between 40°C and 120°C.

10. The method according to any of the preceding claims, **characterized in that** the heating element (4, 4a) is switched off when an upper limit temperature of the heating element (4, 4a) is exceeded, wherein the upper limit temperature is between 120°C and 240°C, or when the determined temperature change rate ($\Delta T$) exceeds a second switch-off change rate ($\Delta AS2$) of the temperature, wherein the second switch-off change rate ($\Delta AS2$) of the temperature is between 5 K/s and 12 K/s, wherein feeding is continued continuously or discontinuously.

11. The method according to claim 10, **characterized in that** the heating element (4, 4a) is switched on when the temperature falls below a lower limit temperature, wherein the lower limit temperature lies between 0 K and 80 K below the upper limit temperature.

12. The method according to claim 11, **characterized in that** on renewed exceedance of the upper limit temperature or on renewed exceedance of the second switch-off change rate ($\Delta AS2$) of the temperature the method is stopped directly, or the steps of switching on the heating element when the temperature falls below the lower limit temperature and switching off the heating element on exceedance of the upper limit temperature or exceedance of the second switch-off change rate ($\Delta AS2$) of the temperature are repeated once or several times, and the method then is stopped.

13. The method according to any of the preceding claims, **characterized in that** an initial feeding of feed water into the chamber (2) or into the steam generator is started when during a first evacuation phase of the chamber (2) the pressure in the chamber (2) falls below a specified pressure and/or when the heating element (4, 4a) reaches a specified control temperature, wherein the specified control temperature is between 40°C and 100°C, and wherein the initial feeding is terminated when the determined temperature change rate ($\Delta T$) reaches a specified third switch-off change rate ($\Delta AS3$) of the temperature.

14. A steam sterilizer (1) comprising a chamber (2) for receiving feed water or comprising a steam generator connected with a chamber (2) for receiving feed water, wherein the steam sterilizer (1) includes a heating element (4, 4a) for heating the feed water, **characterized in that** the steam sterilizer (1) is provided and adapted to carry out a method according to any of claims 1 to 13 in operation.

15. The steam sterilizer (1) according to claim 14, **characterized in that** the steam sterilizer (1) comprises software which, when executed on a processor of the steam sterilizer (1), causes the processor to carry out a method according to any of claims 1 to 13.

**Revendications**

1. Procédé de régulation du niveau d'eau d'alimentation dans une chambre (2) d'un stérilisateur à vapeur (1) ou dans un générateur de vapeur auquel est reliée une chambre (2) d'un stérilisateur à vapeur (1), l'eau d'alimentation étant chauffée au moyen d'un élément chauffant (4, 4a) et de la vapeur d'eau étant ainsi produite pour la chambre (2), **caractérisé en ce qu'**un taux de variation de température ($\Delta T$) de l'élément chauffant (4, 4a) est déterminé et qu'une réalimentation en eau d'alimentation est effectuée en dépendance du taux de variation de température ($\Delta T$) déterminé de l'élément chauffant (4, 4a).

2. Procédé selon la revendication 1, **caractérisé en ce que** le début de la réalimentation est déclenché en dépendance du taux de variation de température ($\Delta T$) de l'élément chauffant (4, 4a) qui a été déterminé.

3. Procédé selon l'une quelconque des revendications précédentes, **caractérisé en ce que** le début de la réalimentation est déclenché lorsque le taux de variation de température ($\Delta T$) déterminé dépasse un taux de variation de mise en marche ($\Delta ES$) prédéterminé.

4. Procédé selon la revendication 3, **caractérisé en ce que** le taux de variation de mise en marche prédéterminé ($\Delta ES$) comprend une valeur de mise en marche fixe, la valeur de mise en marche fixe étant comprise entre 0,5 K/s et 5 K/s.

5. Procédé selon l'une quelconque des revendications précédentes, **caractérisé en ce que** la fin de la réalimentation est déclenchée en dépendance du taux de variation de température ($\Delta T$) déterminé de l'élément chauffant (4, 4a) et/ou en dépendance d'un taux de variation de pression ($\Delta p$) déterminé, le taux de variation de pression ($\Delta p$) étant un taux de variation de pression ($\Delta p$) déterminé dans la chambre (2).

6. Procédé selon la revendication 5, **caractérisé en ce que** la fin de la réalimentation est déclenchée lorsque le taux de variation de température ($\Delta T$) déterminé de l'élément chauffant (4, 4a) est inférieur à un premier taux de variation de désactivation ($\Delta AS1$) prédéterminé de la température, le premier taux de changement de désactivation prédéterminé ($\Delta AS1$) de la température comprenant une valeur de désactivation fixe de la température, la valeur de désactivation fixe de la température étant comprise entre -5K/s et 2K/s.

7. Procédé selon la revendication 5, **caractérisé en ce que** la fin de la réalimentation est déclenchée lorsque le taux de variation de pression déterminé ($\Delta p$) est inférieur à un taux de variation de désactivation prédéterminé ($\Delta ASp$) de la pression ou lorsque la fin de la réalimentation est déclenchée, lorsque le taux de variation de pression déterminé ($\Delta p$) dépasse un taux de variation de désactivation prédéterminé ($\Delta ASp$) de la pression, le taux de variation de désactivation prédéterminé ($\Delta ASp$) de la pression comprenant une valeur de désactivation fixe de la pression, la valeur de désactivation fixe de la pression étant comprise entre -5 mbar/s et +5 mbar/s.

8. Procédé selon l'une quelconque des revendications précédentes, **caractérisé en ce que**, lorsque la fin de la réalimentation est déclenchée, la réalimentation est poursuivie pendant une durée de réalimentation supplémentaire prédéterminée et n'est terminée qu'après l'écoulement de la durée de réalimentation supplémentaire, la durée de réalimentation supplémentaire prédéterminée étant déterminée par un intervalle de temps prédéterminé ou étant déterminée en dépendance de la durée entre le déclenchement du début de la réalimentation et le déclenchement de la fin de la réalimentation.

9. Procédé selon l'une quelconque des revendications précédentes, **caractérisé en ce que** le taux de variation de température ($\Delta T$) de l'élément chauffant (4, 4a) est déterminé en continu, la détermination en continu du taux de variation de température ($\Delta T$) commençant lorsqu'une température prédéterminée de l'élément chauffant est atteinte, la température prédéterminée étant comprise entre 40°C et 120°C.

10. Procédé selon l'une quelconque des revendications précédentes, **caractérisé en ce que** l'élément chauffant (4, 4a) est désactivé lorsqu'une température limite supérieure de l'élément chauffant (4, 4a) est dépassée, ladite température limite supérieure étant comprise entre 120°C et 240°C, ou lorsque le taux de variation de température déterminé ($\Delta T$) dépasse un deuxième taux de variation de désactivation ($\Delta AS2$) de la température, ledit deuxième taux de variation de désactivation ($\Delta AS2$) de la tempéra-

ture étant compris entre 5 K/s et 12 K/s, l'alimentation étant poursuivie de manière continue ou discontinue.

11. Procédé selon la revendication 10, **caractérisé en ce que** l'élément chauffant (4, 4a) est mis en marche lorsqu'une température limite inférieure n'est pas atteinte, la température limite inférieure étant comprise entre 0 K et 80 K en dessous de la température limite supérieure.

12. Procédé selon la revendication 11, **caractérisé en ce que**, en cas de nouveau dépassement de la température limite supérieure ou en cas de nouveau dépassement du deuxième taux de variation de désactivation ($\Delta AS2$) de la température, le procédé est directement interrompu, ou les étapes de mise en marche de l'élément chauffant en cas de sous-dépassement de la température limite inférieure et de la désactivation de l'élément chauffant en cas de dépassement de la température limite supérieure ou de dépassement du deuxième taux de variation de désactivation ($\Delta AS2$) de la température sont répétées une ou plusieurs fois et le procédé est alors interrompu.

13. Procédé selon l'une quelconque des revendications précédentes, **caractérisé en ce qu'**une alimentation initiale d'eau d'alimentation dans la chambre (2) ou dans le générateur de vapeur est démarrée lorsque, pendant une première phase d'évacuation de la chambre (2), une pression prédéterminée dans la chambre (2) n'est pas atteinte et/ou lorsque l'élément chauffant (4, 4a) atteint une température de régulation prédéterminée, la température de régulation prédéterminée étant comprise entre 40°C et 100°C, et l'alimentation initiale étant terminée lorsque le taux de variation de température déterminé ($\Delta T$) atteint un troisième taux de variation de désactivation prédéterminé ($\Delta AS3$) de la température.

14. Stérilisateur à vapeur (1) comprenant une chambre (2) destinée à recevoir de l'eau d'alimentation ou un générateur de vapeur relié à une chambre (2) destinée à recevoir de l'eau d'alimentation, le stérilisateur à vapeur (1) comprenant un élément chauffant (4, 4a) destiné à chauffer l'eau d'alimentation, **caractérisé en ce que** le stérilisateur à vapeur (1) est prévu et adapté pour exécuter, en fonctionnement, un procédé selon l'une quelconque des revendications 1 à 13.

15. Stérilisateur à vapeur (1) selon la revendication 14, **caractérisé en ce que** le stérilisateur à vapeur (1) comprend un logiciel qui, lorsqu'il est exécuté sur un processeur du stérilisateur à vapeur (1), amène le processeur à exécuter un procédé selon l'une quelconque des revendications 1 à 13.

FIG 1

FIG 2

FIG 3

FIG 4

FIG 5

FIG 6

**EP 4 431 119 B1**

**IN DER BESCHREIBUNG AUFGEFÜHRTE DOKUMENTE**

**In der Beschreibung aufgeführte Patentdokumente**

- DE 10260895 A1
- DE 102009044053 A1
- EP 383327 A1
- EP 193863 A2
- EP 1108384 B1
- DE 60004509 T2
- EP 3276068 A1